# FASCICULE DE BREVET EUROPEEN

(11) **EP 3 526 244 B1**
(45) Date de publication et mention de la délivrance du brevet: **06.09.2023**
(21) Numéro de dépôt: 17794365.1
(22) Date de dépôt: 11.10.2017
(51) Int. Cl.: C07K 14/72, A61K 38/08, A61K 38/10, C07K 7/06, C07K 7/08

(54) **PEPTIDES DÉRIVÉS DU PROPEPTIDE NTSR3 ET LEUR UTILISATION DANS LE TRAITEMENT DE LA DÉPRESSION**
AUS DEM PROPEPTID NTSR3 ABGELEITETE PEPTIDE UND DEREN VERWENDUNG BEI DER BEHANDLUNG VON DEPRESSION
PEPTIDES DERIVED FROM THE PROPEPTIDE NTSR3 AND THEIR USE IN THE TREATMENT OF DEPRESSION

(30) Priorité: 11.10.2016 FR 1659781; 16.03.2017 FR 1752162; 11.05.2017 FR 1754127
(43) Date de publication de la demande: 21.08.2019
(73) Titulaire: Centre National de la Recherche Scientifique, 75016 Paris (FR); Université de Nice Sophia Antipolis, 06100 Nice (FR)
(72) Inventeur: MAZELLA, Jean, 06410 Biot (FR); BORSOTTO, Marc, 06130 Grasse (FR); HEURTEAUX, Catherine, 06480 La-Colle-Sur-Loup (FR); DJILLANI, Alaeddine, Aïn-Oulmene Setif 19200 (DZ); MORENO, Sébastien, 06140 Vence (FR); PIETRI, Mariel, 20000 Ajaccio (FR)
(74) Mandataire: Lavoix
(86) Numéro de dépôt international: PCT/FR2017/052788
(87) Numéro de publication internationale: WO 2018/069641

(56) Documents cités:
- WO-A1-2009/103898
- WO-A2-2004/056385
- WO-A2-2015/110915
- US-A1- 2012 322 060
- "LICENSING OPPORTUNITIES CNRS patent portfolio related to Central Nervous System Disorders", , 1 janvier 2014 (2014-01-01), XP055191985, Extrait de l'Internet: URL:http://www.fist.fr/wp-content/uploads/ file/Portefeuilles/CNRS [extrait le 2015-05-28]
- DEVADER CHRISTELLE ET AL: "Serum sortilin-derived propeptides concentrations are decreased in major depressive disorder patients", JOURNAL OF AFFECTIVE DISORDERS, ELSEVIER BIOCHEMICAL PRESS, AMSTERDAM, NL, vol. 208, 4 novembre 2016 (2016-11-04), pages 443-447, XP029835021, ISSN: 0165-0327, DOI: 10.1016/J.JAD.2016.10.049

## Description

### Domaine technique de l'invention

La présente invention concerne des peptides courts dérivés du récepteur 3 de la neurotensine (NTSR3) et à leur utilisation dans différentes pathologies (diabète, cancer, épilepsie, AVC, pathologies neurodégénératives, etc...), notamment dans le traitement de pathologies psychiatriques, en particulier de patients souffrant ou ayant souffert de dépression, par exemple après un AVC. La présente invention se rapporte en particulier à l'utilisation de ces peptides pour la fabrication d'un médicament, par exemple un antidépresseur.

La présente invention trouve une application dans les secteurs de l'industrie pharmaceutique et notamment dans les domaines de développement du médicament utilisé dans le traitement de différentes pathologies (diabète, cancer, AVC, épilepsie, maladies psychiatriques, pathologies neurodégénératives, etc...).

Dans la description ci-dessous, les références entre crochets (**[ ]**) renvoient à la liste des références présentée à la fin du texte.

### État de la technique

La dépression représente un problème majeur de santé publique. Les travaux les plus récents ont confirmé la forte prévalence de la dépression : sur leur vie entière, 20% des femmes et 10% des hommes ont fait, font ou feront un épisode dépressif **[1].** De tels chiffres sont à l'évidence marquant; ils le sont plus encore lorsqu'on s'intéresse à la complication majeure de la dépression, le suicide, qui se chiffre à 12000 décès par an dans des pays comme la France **[2].**

La dépression est une maladie très fréquente et souvent handicapante. Elle peut toucher jusqu'à 20% de la population dans les pays industrialisés. Ses origines sont diverses et multiples. Cette pathologie affecte aussi bien le psychisme que le comportement et la physiologie des patients. Les traitements de la dépression sont également multiples et les mécanismes d'action des médicaments utilisés ne sont pas clairement établis.

L'organisation mondiale de la santé (OMS) prévoit que la dépression unipolaire sera la deuxième cause de handicap en 2020. A la souffrance personnelle et familiale que représente la dépression s'ajoute le poids social important de cette pathologie. La dépression représente déjà l'une des premières causes d'arrêt du travail, avec une charge économique qui s'élève à plus de 30 milliards d'euros par an. Malgré l'arsenal thérapeutique mis à disposition du corps médical, en particulier les SSRI (« *sélective serotonin reuptake inhibitors* ») et SNRI (« *serotonin norepinephrine reuptake inhibitors* »), 30% de la population dépressive n'a pas de traitement. Par ailleurs, le délai d'action des antidépresseurs est de l'ordre de 3 à 6 semaines et les effets secondaires sont souvent importants.

D'une façon générale, on estime que 15% des patients déprimés décèdent par suicide. Chez la plupart des malades, la dépression est due à l'interaction entre une prédisposition génétique et des facteurs environnementaux comme le stress ou les traumatismes émotionnels **[3].** La maladie est fréquente et le marché des antidépresseurs (AD) est immense (au moins 10 milliards d'euros par an).

Néanmoins, si ces antidépresseurs améliorent l'état des patients dans environ 70% des cas, ils n'entraînent une rémission complète de la maladie que chez 30 à 40% d'entre eux. De plus, près d'un tiers des sujets traités résiste aux traitements existants. Cet état de fait oblige donc à envisager de nouveaux traitements, capables de prendre en compte les mécanismes de la dépression **[3].**

Dans l'arsenal thérapeutique mis à disposition du corps médical, les antidépresseurs tricycliques (TCA) avec l'amitriptyline et l'imipramine ont été les premiers découverts, suivis par les inhibiteurs de la monoamine oxydase (IMAO), irréversibles et non sélectifs comme la phénelzine et la pargyline. Les effets indésirables, en particulier la cardiotoxicité des TCA (surtout en cas de surdosage) et les crises hypertensives des IMAO (interactions avec la tyramine alimentaire, le fameux « *cheese effect* ») ont poussé la recherche vers de nouvelles molécules d'efficacité thérapeutique identique mais de meilleure acceptabilité.

La notion de sélectivité est alors apparue avec les inhibiteurs spécifiques de la recapture de la noradrénaline (NA) ou de la sérotonine (5-hydroxytryptamine ou 5HT). Les essais cliniques de phase III ont démontré pour ces nouvelles molécules une efficacité équivalente aux antidépresseurs de première génération et une meilleure sécurité, notamment en cas de surdosage.

Les inhibiteurs spécifiques de la recapture de la sérotonine (ISRS) et les inhibiteurs spécifiques de la recapture de la noradrénaline (ISRN) sont actuellement les molécules les plus utilisées **[4-5].** Les AD sont ainsi le plus souvent associés à une facilitation de la transmission des systèmes monoaminergiques.

Bien que la sérotonine, la noradrénaline et la dopamine sont impliquées de façon certaine, il est admis aujourd'hui que les modifications des taux de monoamines produites par les AD et les processus adaptatifs qui en découlent, en particulier l'altération de la sensibilité de certains de leurs récepteurs, ne peuvent expliquer à eux seuls le mécanisme d'action des antidépresseurs.

Ainsi, il est difficile de corréler le délai de 3 à 6 semaines nécessaire à l'obtention des AD avec l'augmentation des taux synaptiques de monoamines, qui intervient dès la première administration du produit. En près d'un demi-siècle, le nombre d'hypothèses sur la pathogénèse de la dépression et son traitement n'a cessé d'évoluer.

Par exemple, des concentrations élevées de glucocorticoïdes sont généralement associées à un effet négatif de l'humeur, ainsi que des altérations structurelles de l'hippocampe, par l'intermédiaire d'une diminution de la synthèse de BDNF (« *brain-derivated neurotrophic factor*»), d'une sécrétion excessive d'acide glutamique et/ou d'une diminution de la capture du glucose **[6].**

Conformément à ces observations, des inhibiteurs de la synthèse des glucocorticoïdes et des antagonistes des récepteurs de glucocorticoïdes exercent des effets de type AD **[7].**

Des antagonistes agissant sur les récepteurs de la substance P, en particulier le NK1, ou le récepteur CRF (« *corticotropin-releasing factor*»), ainsi que des antagonistes des récepteurs du NMDA ont été développés avec une certaine efficacité **[8-10].**

Diverses études récentes réalisées dans des situations de stress et des modèles de dépression ont impliqué la neurogenèse dans l'étiologie des troubles dépressifs majeurs **[11-13].** Il a été démontré que tous les traitements chroniques AD, y compris l'électrochoc, stimulent la prolifération des cellules pro génitrices à l'origine des neurones de la couche granulaire de l'hippocampe.

On sait également que les AD modulent l'expression de différents facteurs impliqués dans la survie et la croissance des cellules, tels que la CREB, le Bcl2 ou les MAP-kinases. Toutefois, l'importance fonctionnelle de ces neurones néoformés dans la physiopathologie des troubles de l'humeur reste controversée **[14].**

L'ensemble de ces indications montrent que la dépression est une maladie complexe dont la physiopathologie est multifactorielle et, en conséquence, le traitement d'une telle pathologie reste un challenge.

Depuis plus de quarante ans, la recherche sur la dépression et le développement de médicaments efficaces a été dominée par l'hypothèse monoaminergique. Bien que les neurotransmetteurs monoaminergiques (sérotonine, noradrénaline et dopamine) sont impliqués de façon indiscutable, le nombre d'hypothèses sur la physiopathologie de la dépression et les mécanismes d'action des AD n'a cessé d'évoluer.

Les médicaments AD utilisés aujourd'hui produisent une gamme d'effets indésirables, parmi lesquels la sécheresse de la bouche, la vision floue et l'altération de la fonction intestinale (diarrhée ou constipation). Même si beaucoup d'effets secondaires sont passagers (comme la nausée), certains semblent être constants au fil du temps (comme les effets sexuels) et risquent d'affecter l'assiduité au traitement à long terme. C'est la raison pour laquelle la recherche de nouvelles molécules agissant sur des récepteurs ou canaux nouvellement identifiés dans la dépression est cruciale.

Certaines protéines, récepteurs et canaux, ont été impliqués dans les mécanismes moléculaires de la dépression. C'est notamment le cas du récepteur 3 de la neurotensine (NTSR3), également appelé sortiline, dont l'inactivation chez la souris produit un phénotype de résistance à la dépression, analogue à celui qui a été observé chez des souris invalidées pour le canal potassique de fond TREK-1.

L'étude récente STAR*D (Sequenced Treatment Alternatives to Relieve Depression) **[15],** a identifié de manière pharmacogénétique TREK-1 comme le gène impliqué dans la réponse antidépressive chez l'homme. Cette étude suggère également l'utilité d'un modèle animal pour la recherche de traitements antidépresseurs dans l'identification de gènes candidats pour une étude chez l'homme. Des bloqueurs des canaux TREK-1 représentent donc un nouveau concept dans le domaine de la conception de médicaments antidépresseurs.

A ce jour, une molécule interagissant efficacement avec les canaux TREK-1 a été identifiée. Il s'agit d'un peptide de 44 acides aminés, appelé propeptide (PE) : QDRLDAPPPPAAPLPRWSGPIGVSWGLRAAAAGGAFPRGGRWRR (SEQ ID NO :°1) libéré lors de la maturation par la furine du récepteur 3 de la neurotensine (NTSR3) qui devient un ligand de ce même récepteur. De façon intéressante, il a été montré que ce propeptide est également capable de bloquer l'activité du canal TREK-1. Il a été montré qu'une partie de 17 acides aminés dérivés dudit propeptide, appelée spadine (YAPLPRWSGPIGVSWGLR, SEQ ID NO :°2), porte toute l'activité de liaison sur le récepteur **[16].** Il a récemment été mis en évidence que la spadine bloque spécifiquement les canaux TREK-1. Elle se comporte comme un antidépresseur dans des modèles de rongeurs de la dépression, sans provoquer d'effets secondaires sur les autres fonctions de ce canal et les autres canaux potassiques ni au niveau cardiaque et sur l'index glycémique **[17].** Il a également été récemment mis en évidence un rôle de la spadine dans la régulation de la synaptogenèse **[18].** WO2015110915 divulgue une liste d'analogues de la spadine et leur application thérapeutique.

Il existe donc un réel besoin de nouvelles molécules utilisables pour le traitement de la dépression, molécules plus efficaces, mieux tolérées et ayant une action plus rapide.

### Description de l'invention

La présente invention a précisément pour rôle de répondre au besoin et de résoudre les inconvénients de l'art antérieur.

Dans ce but les Inventeurs ont recherché si de plus petits peptides résultant de la dégradation de la spadine pourraient aussi avoir un rôle d'antidépresseur, et ont identifié de manière tout à fait inattendue de nouveaux peptides actifs dérivés du propeptide (PE) modifié ou non.

Le peptide PE(22-28) de séquence SEQ ID NO : 3 (GVSWGLR) dérivé également dudit propeptide a été utilisé comme peptide de base pour la conception d'autres peptides: peptide PE(22-28) biotinylé, le peptide PI-PE(22-28) de séquence SEQ ID NO : 6 (PIGVSWGLR), le peptide PI-PE(22-28) biotinylé, le peptide G/A-PE(22-28) de séquence SEQ ID NO : 7 (AVSWGLR) correspondant à la séquence SEQ ID NO : 3 dans laquelle la glycine en position 22 a été remplacée par une alanine, le peptide G/A-PE(22-28) biotinylé, le peptide dansyl-PE(22-28) correspondant à la séquence SEQ ID NO : 3 dans laquelle un groupement chimique dansyle a été ajouté en N-terminal, les peptide O-méthyl-PE(22-28) et O-éthyl-PE(22-28) correspondant à la séquence SEQ ID NO : 3 dans laquelle un groupement chimique O-méthyl ou O-éthyl a été ajouté en C-terminal, respectivement.

Ces analogues PE(22-28), appelés ci-après analogues de la spadine, présentent une importante action antidépresseur (AD) dans les souris, comme cela a été mesuré par des tests comportementaux (test de la nage forcée, test de la résignation apprise), et induisent rapidement et plus efficacement une neurogenèse et une synaptogenèse. Du fait que les cibles physiologiques de ces analogues de la spadine sont les mêmes que celles de la spadine, leurs mécanismes d'action sont très similaires de ceux de la spadine. Ces analogues de la spadine agissent par l'inhibition du canal TREK-1 avec une meilleure affinité (x333) que la spadine elle-même (0,12 nM pour les analogues de la spadine vs 40 nM pour la spadine). Compte-tenu de la meilleure affinité et de la plus petite taille des analogues de la spadine, les principaux avantages de ces peptides sont premièrement une diminution de la quantité de produit à administrer d'où une diminution des effets secondaires potentiellement associés (toxicité, tumorégénicité, etc...), et deuxièmement une diminution du coût de production ainsi que sa disponibilité pour n'importe quelle voie d'administration connue (icv, iv, ip et orale). Il a été précédemment mis en évidence que la spadine a un effet rapide, 4 jours au lieu de 21 jours pour les antidépresseurs classiques. Ainsi l'utilisation de peptides courts étant à la fois rapidement disponibles et rapidement éliminés de l'organisme représente une garantie d'un produit thérapeutique efficace sans effets secondaires; ce qui est d'une importance cruciale en santé publique.

La présente divulgation décrit ainsi un peptide dérivé du propeptide (PE) du récepteur 3 de la neurotensine (NTSR3) choisi parmi le peptide PE(22-28) biotinylé correspondant à la séquence SEQ ID NO : 3 biotinylée, le peptide PI-PE(22-28) de séquence SEQ ID NO : 6 (PIGVSWGLR), le peptide PI-PE(22-28) biotinylé correspondant à la séquence SEQ ID NO : 6 biotinylée, le peptide G/A-PE(22-28) de séquence SEQ ID NO : 7 (AVSWGLR) correspondant à la séquence SEQ ID NO : 3 dans laquelle la glycine en position 22 a été remplacée par une alanine, le peptide G/A-PE(22-28) biotinylé correspondant à la séquence SEQ ID NO : 7 biotinylée, le peptide dansyl-PE(22-28) correspondant à la séquence SEQ ID NO : 3 dans laquelle un groupement chimique dansyle a été ajouté en N-terminal, et les peptides O-méthyl-PE(22-28) et O-éthyl-PE(22-28) correspondant à la séquence SEQ ID NO : 3 dans laquelle un groupement chimique O-méthyl ou O-éthyl a été ajouté en C-terminal, respectivement.

La présente invention est définie par les revendications annexées.

La présente invention a ainsi pour objet un peptide dérivé du propeptide (PE) du récepteur 3 de la neurotensine (NTSR3) choisi parmi le peptide PE(22-28) biotinylé correspondant à la séquence SEQ ID NO : 3 biotinylée, le peptide Pl-PE(22-28) de séquence SEQ ID NO : 6 (PIGVSWGLR), le peptide PI-PE(22-28) biotinylé correspondant à la séquence SEQ ID NO : 6 biotinylée, le peptide G/A-PE(22-28) de séquence SEQ ID NO : 7 (AVSWGLR) correspondant à la séquence SEQ ID NO : 3 dans laquelle la glycine en position 22 a été remplacée par une alanine, le peptide G/A-PE(22-28) biotinylé correspondant à la séquence SEQ ID NO : 7 biotinylée, le peptide dansyl-PE(22-28) correspondant à la séquence SEQ ID NO : 3 dans laquelle un groupement chimique dansyle a été ajouté en N-terminal, et les peptides O-méthyl-PE(22-28) et O-éthyl-PE(22-28) correspondant à la séquence SEQ ID NO : 3 dans laquelle un groupement chimique O-méthyl ou O-éthyl a été ajouté en C-terminal, respectivement.

La présente invention a également pour objet une séquence d'acide nucléique codant pour un peptide de l'invention. Cette séquence est de préférence utilisable pour fabriquer le peptide de la présente invention ou un fragment ou dérivé de celui-ci par transfection. Il s'agit par exemple de la séquence avec les numéros d'accession GenBank pour les ARNmessagers de la sortiline SORT 1, chez la souris : NM_019972, chez l'Homme : NM_002959.

La présente invention a également pour objet un vecteur comprenant une séquence d'acide nucléique selon l'invention. Il peut s'agir de tout vecteur approprié pour la transformation d'une cellule hôte en vue de faire fabriquer par ladite cellule, par une technique de recombinaison génétique, un peptide de la présente invention. Le vecteur peut être obtenu à partir d'un vecteur choisi, par exemple, dans le groupe comprenant pIRES et pIRES2 et leurs dérivés, pcDNA3 et ses dérivés, pGEX et ses dérivés.

La présente invention a également pour objet une cellule hôte comprenant un peptide selon la présente invention et/ou une séquence d'acide nucléique selon la présente invention et/ou un vecteur selon la présente invention. La cellule hôte peut être toute cellule appropriée pour être transformée et fabriquer ledit peptide de l'invention ou fragment ou dérivé de celui-ci. Il peut s'agir par exemple de cellules COS-7, HEK 293 et dérivées, N1 E115 et apparentées.

La présente invention se rapporte donc également à un procédé de production d'un peptide selon l'invention comprenant les étapes suivantes :
- transfecter une cellule hôte avec un acide nucléique selon l'invention ou transformer une cellule hôte avec un vecteur de l'invention ;
- cultiver ladite cellule hôte dans des conditions permettant l'expression d'un peptide selon l'invention ; et
- récupérer ledit peptide.

Les techniques de transfection et de transformation utilisables pour fabriquer un peptide de la présente invention sont celles connues de l'homme du métier, par exemples celles décrites dans Alloui A et al, EMBO J. 2006 [19].

Selon l'invention, compte-tenu de la petite taille des peptides de l'invention, le procédé de fabrication préféré du peptide est la synthèse chimique, par exemple en phase solide. Toute technique connue de l'homme du métier peut être utilisée. Par exemple, le peptide peut être synthétisé selon la technique en phase solide de Krieger DE et al, Proc Nat Acad Sci U.S.A. 1976 **[20].**

Les peptides de l'invention ouvrent une nouvelle voie dans la mise en place d'une nouvelle classe d'antidépresseurs et de nouvelles stratégies thérapeutiques de la dépression.

La présente invention a également pour objet un peptide selon l'invention pour une utilisation comme médicament, en particulier comme antidépresseur.

La présente invention a également pour objet un peptide selon l'invention pour une utilisation dans le traitement de différentes pathologies telles que le diabète, le cancer, l'AVC, les pathologies neurodégénératives, les pathologies psychiatriques, notamment la dépression, par exemple la dépression post-AVC. Ces hypothèses sont supportées par plusieurs faits expérimentaux. Les canaux TREK-1 sont présents à la surface des cellules bêta-pancréatiques telles que les cellules bêta-TC3. De plus, il a été démontré que la spadine (YAPLPRWSGPIGVSWGLR, SEQ ID NO :°2) augmente la sécrétion d'insuline à la suite d'une hyperglycémie (Hivelin C et al., J Diabètes Res. 2016) **[27].** En outre, sur les cellules βTC3, les inventeurs ont mis en évidence que le peptide PE(22-28) de séquence SEQ ID NO : 3 : GVSWGLR bloque les courants potassiques sensibles à l'acide arachidonique (aa). Dans la mesure où la spadine, le peptide PE(22-28), et les peptides de la présente invention ont les mêmes cibles physiologiques, de fait ces peptides peuvent également être considérés comme des molécules hypoglycémiantes. D'autre part, les canaux TREK-1 sont également présents à la surface des cellules cancéreuses telles que les cellules LNCaP (cancer de la prostate). Sur les cellules LNCaP, les inventeurs ont mis en évidence que le peptide PE(22-28) bloque les courants potassiques sensibles à l'acide arachidonique (aa) et inhibe leur croissance. Le peptide PE(22-28) et les peptides de la présente invention ont les mêmes cibles physiologiques, de fait peuvent également être considérés comme présentant une activité anticancéreuse. L'utilisation d'un peptide selon l'invention peut également être envisagée dans la neuroprotection vis-à-vis d'un AVC, ou de l'épilepsie.

Les peptides de la présente invention tels que définis ci-dessus, à l'exception du G/A-PE(22-28), sont des produits naturels qui constituent un nouveau type d'antidépresseur. Ils permettent d'éviter tous les effets secondaires non désirables des médicaments actuellement utilisés pour le traitement de la dépression. Les effets secondaires des médicaments utilisés dans l'art antérieur proviennent de leur nature chimique. Ces médicaments de l'art antérieur sont des molécules qui ont souvent la propriété de passer les membranes plasmiques cellulaires de manière passive, ce qui entraîne des interactions non spécifiques avec des effecteurs intracellulaires. Le caractère peptidique de la présente invention permet d'éviter ce problème.

Par ailleurs, l'action directe d'un peptide de la présente invention sur le canal potassique TREK-1 pourrait bien diminuer les délais d'action, souvent longs d'un antidépresseur classique.

D'autres avantages pourront apparaître à l'homme du métier à la lecture des exemples qui suivent, donnés bien entendu à titre illustratif et non-limitatif en référence aux figures annexées.

### Brève description des figures

- La figure 1 représente l'action des analogues de la spadine de l'invention sur l'activité du canal TREK-1, en comparaison avec celle de la spadine et du peptide PE(22-28), mesurée *in vitro* par des expérimentations électrophysiologiques.
- La figure 2 représente l'action des analogues de la spadine G/A-PE(22-28) et G/A-PE(22-28) biotinylé sur l'activité du canal TREK-1, en comparaison avec celle de la spadine et du peptide pE(22-28), mesurée *in vitro* par des expérimentations électrophysiologiques.
- La figure 3 représente l'activité antidépresseur des analogues de la spadine selon la présente invention, en comparaison avec celle de la spadine et du peptide PE(22-28), mesurée *in vivo* chez des souris soumises au test de la nage forcée (FST).
- La figure 4 représente l'activité antidépresseur des analogues de la spadine G/A-PE(22-28) et G/A-PE(22-28) biotinylé, en comparaison avec celle de la spadine et du peptide PE(22-28), mesurée *in vivo* chez des souris soumises au test de la résignation apprise (LHT).
- La figure 5 représente l'étude par immunohistochimie de l'action des analogues de la spadine G/A-PE(22-28) et G/A-PE(22-28) biotinylé, en comparaison de celle du peptide PE(22-28), sur la neurogenèse.
- La figure 6 représente l'étude de l'action des analogues de la spadine G/A-PE(22-28) et G/A-PE(22-28) biotinylé (B et C, respectivement), en comparaison de celle du peptide PE(22-28) (A), sur la synaptogenèse. (D) histogramme de PSD-95 pour chaque peptide.
- La figure 7 représente l'action du peptide PE(22-28) sur l'activité du canal TREK-1 exprimé dans les cellules pancréatiques βTC3 (A) et cancéreuses LNCaP (B), mesurée *in vitro* par des expérimentations électrophysiologiques, ainsi que sur la croissance des cellules cancéreuses LNCaP (C).

### EXEMPLES

### EXEMPLE 1 : ACTION DES ANALOGUES DE LA SPADINE SUR L'ACTIVITÉ DU CANAL TREK-1

Toutes les expériences de mesure de l'effet des analogues de la spadine et de la spadine ont été réalisées sur des cellules bêta-TC3 ou LNCaP ou HEK293 transfectées de façon stable avec le canal TREK-1, hTREK-1/HEK293. La lignée cellulaire hTREK-1/HEK293 a été préparée au laboratoire **[21].** Ces cellules expriment également un gène rapporteur : E-Green Fluorescent protein (EGFP) permettant leur visualisation.

Ces cellules ont été ensemencées à une densité de 20 000 cellules / boîte de 35mm de diamètre, 24 à 72 heures avant d'être utilisées.

Les cellules HEK293 ont été cultivées dans un milieu DMEM (Gibco) / 10% sérum de veau fétal (SVF, ICN) / 1% d'un mélange streptomycine + pénicilline (Gibco) / 1 % glutamax (Gibco)/ 0,5 mg/mL de G418 (Sigma) à 37°C en présence de 5% de CO₂.

Les cellules bêta-TC3 ont été cultivées dans un milieu RPMI 1640 supplementé par 2.5% SVF, 50 µM bêta-mercaptoethanol, 10 mM HEPES, 1 mM pyruvate de Sodium, 50 µg/ml gentamycine et 1% d'un mélange streptomycine + pénicilline à 37°C en présence de 5% de CO₂.

Les cellules LNCaP ont été cultivées dans un milieu DMEM (Gibco) / 10% sérum de veau fétal (SVF, ICN) / 1% d'un mélange streptomycine + pénicilline (Gibco) / 1 % glutamax (Gibco)/ à 37°C en présence de 5% de CO₂.Au jour 1, les cellules hTREK-1/HEK293 ou bêta-TC3 ou LNCaP ont été ensemencées à 20 000 cellules par boîte de diamètre 35mm contenant 2 ml de milieu de culture. Du jour 2 au jour 4, les cellules ont été mesurées par des méthodes électrophysiologiques.

Toutes les mesures ont été faites à température ambiante, c'est-à-dire à 21 - 22°C. Les cellules hTREK-1/HEK293 ont été repérées grâce à la fluorescence émise par la EGFP après excitation à 480nm.

La technique du patch clamp sur cellule entière a été utilisée pour mesurer l'activité des canaux TREK-1. L'appareillage utilisé est le RK 400 patch-clamp amplifier (Axon Instruments, U.S.A.). Les pipettes de patch de résistance 1,3 à 8 MΩ ont été préparées à partir de capillaires en verre borosilicate et ont été remplies avec une solution 155 mM KCI, 3 mM MgCl₂, 5 mM EGTA, 10 HEPES/KOH pH 7,2.

Le milieu de culture cellulaire a été remplacé par une solution 150 mM NaCl, 5 mM KCI, 3 mM MgCl₂, 1mM CaCl₂, 10 mM HEPES/ NaOH pH 7,4 contenant 10 mM Chlorure de TétraEthyl Ammonium, 3 mM de 4-Aminopyridine. Les cellules ont été perfusées en continu avec cette solution. Le potentiel de repos de la membrane de la cellule mesurée a été fixé à -80mV.

Les variations de voltage ont été obtenues soit par rampe continue (de -100 à + 60mV) soit par saut de potentiel de 10mV (de -100 à + 60mV, 1,5 seconde par saut). Les données obtenues ont été analysées avec le logiciel Pclamp.

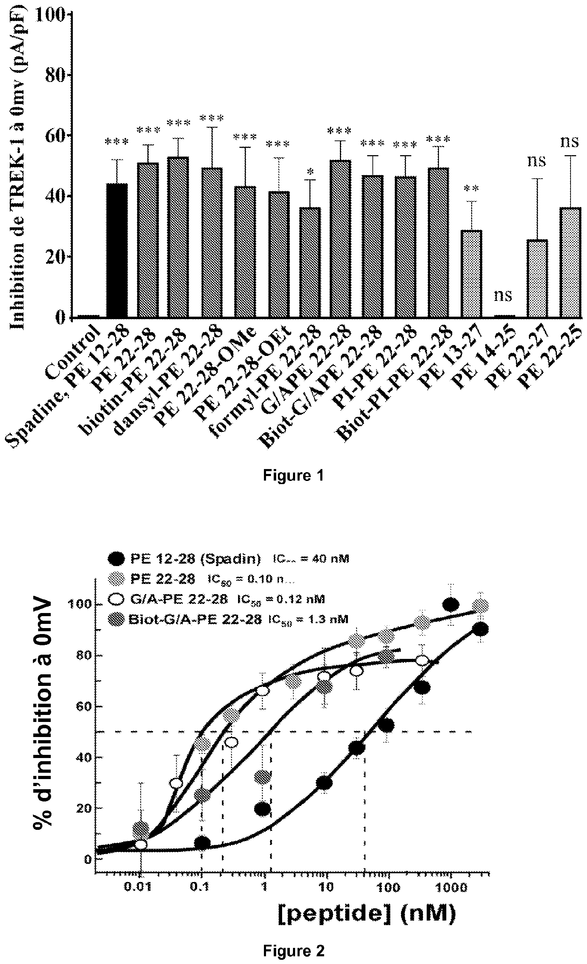

Les courants décrits dans les figures 1 et 2 ont été obtenus à 0 mV. Les résultats des figures 1 et 2 exprimés sont les moyennes ± la déviation standard. La valeur de l'IC₅₀ de la figure 2 a été obtenue en traçant les données expérimentales à l'aide d'une fonction sigmoïdale.

L'activité du canal a ensuite été mesurée par la technique de « *patch-clamp* » en configuration cellule entière comme décrit ci-dessus.

Les inhibitions de l'activité des canaux TREK-1 mesurée à 0 mV de l'ensemble des analogues de la spadine de l'invention et de la spadine, sont représentées sur la figure 1. Elle indique que tous les analogues de la spadine, sauf les peptides PE(14-25), PE(22-25) et PE(22-27), sont capables de bloquer l'activité des canaux TREK-1 dans la même mesure que la spadine (PE(12-28)).

Une courbe dose-réponse de l'inhibition de l'activité des canaux TREK-1 mesurée à 0 mV a été réalisée en présence des analogues G/A-PE(22-28) ou G/A-PE(22-28) biotinylé ou de la spadine. Ces courbes sont représentées sur la figure 2. Elle indique une concentration de demi-effet (IC₅₀) de 0,12 nM et 0,13 nM pour les peptides G/A-PE(22-28) et G/A-PE(22-28) biotinylé, respectivement, et de 40 nM pour la spadine.

L'ensemble des résultats montre que la plupart des analogues de la spadine sont capables de bloquer les canaux TREK-1 avec une meilleure affinité (x333) que la spadine et, pour certains analogues, avec une affinité équivalente de celle du peptide PE(22-28).

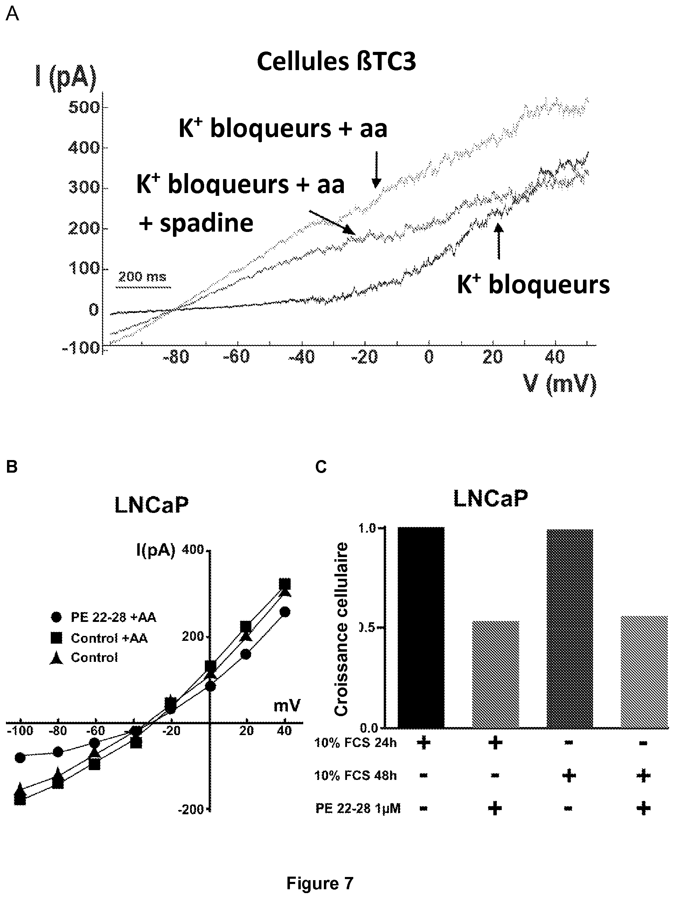

Les intensités du courant mesurées en fonction du voltage [I=f(V)] appliqué sur les cellules βTC3 décrites dans la figure 7A, indiquent que la spadine est capable de bloquer les canaux TREK-1 exprimés sur les cellules βTC3 et préalablement activés avec l'acide arachidonique (aa). La spadine et les peptides de la présente invention ont les mêmes cibles physiologiques, de fait ces peptides peuvent être considérés comme des molécules hypoglycémiantes.

Les intensités du courant mesurées en fonction du voltage [I=f(V)] appliqué sur les cellules LNCaP décrites dans la figure 7B, indiquent que le peptide PE(22-28) est capable de bloquer les canaux TREK-1 exprimés sur les cellules LNCaP et préalablement activés avec l'acide arachidonique (aa). En outre, la croissance des cellules LNCaP obtenue après comptage des cellules en culture après 24 ou 48h, en présence ou en absence du peptide PE(22-28), indiquent que le peptide pE(22-28) est capable d'inhiber la croissance cellulaire. Le peptide PE(22-28) et les peptides de la présente invention ont les mêmes cibles physiologiques, de fait ces peptides peuvent également être considérés comme des molécules présentant une activité anticancéreuse.

### EXEMPLE 2 : TESTS DE COMPORTEMENT

### Test de la nage forcée (ou Forced Swimming Test, FST)

Un test de comportement considéré comme classique a été développé pour déterminer l'activité antidépresseur d'une substance (Nestler EJ et al, 2002 ; Cryan J and Holmes A, 2005) **[22, 23].** Les effets des analogues de la spadine de la présente invention ont été comparés à ceux de la solution saline dans laquelle les analogues de la spadine de l'invention ont été dissous et à ceux de la spadine. La souche de souris utilisée a été la souche C57BI/6J.

La solution saline, les analogues de la spadine et la spadine ont été administrés aux souris par voie intrapéritonéale (IP) à raison d'une dose de 100 µg/kg en un bolus de 100µl.

L'expérience a consisté, 30 min après administration de la substance à tester, à plonger la souris pendant 6 minutes dans un récipient de 15 cm de diamètre, de 30 cm de hauteur contenant 11 cm d'une eau à 22°C et de mesurer le temps d'immobilité lors des 4 dernières minutes.

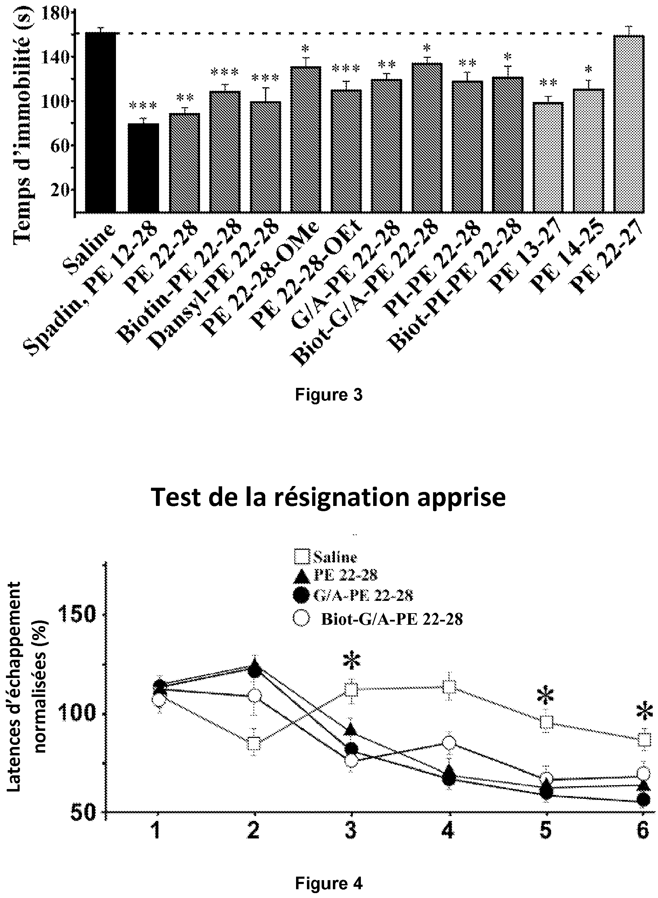

Les résultats de ces tests sont représentés sur la figure 3: Tests FST, les valeurs ± SEM ont statistiquement été comparées à la condition contrôle (sérum physiologique) *** p < 0,001, (Test « ANOVA » = test d'analyse de variances)

Les résultats montrent que les analogues de la spadine capables d'inhiber l'action des canaux TREK-1 sont efficaces dans le test de la nage forcée après leur injection IP dans les souris. En revanche, le peptide PE(22-27) qui n'est pas efficace pour inhiber les canaux TREK-1 est également inefficace pour réduire le temps d'immobilité dans le test de la nage forcée.

### Test de la résignation apprise (ou Learned helplessness test, LHT)

La résignation apprise ou *learned helplessness* est un modèle basé sur une administration répétée de chocs électriques permettant d'induire un état résigné chez l'animal. En effet, il a été montré que les animaux recevant des chocs électriques inévitables, se résignent à les recevoir et n'essayent pas de fuir (Seligman and Beagley, Learned helplessness in the rat. J Comp Physiol Psychol 88, 534-541, 1975) **[24].**

L'appareil est composé des deux compartiments séparés par une porte coulissante et avec un plancher constitué d'une grille métallique par laquelle sont administrées les impulsions électriques. Le protocole de la résignation apprise est divisé en deux étapes, une étape de conditionnement de l'animal et une étape de test.

Le conditionnement s'est fait sur quatre jours pendant lesquels les souris ont reçu 360 chocs électriques non évitables de 0,3 mA, durant une heure (la porte, séparant les deux compartiments, étant fermée). Les chocs étaient d'une durée de 2 secondes et un intervalle de 8 secondes a été respecté entre chaque choc. Cette étape de conditionnement a permis d'induire un phénomène de résignation chez les animaux.

Le jour du test, les souris ont été placées dans les mêmes compartiments mais avec une possibilité d'échapper au choc. Le test a compté 30 essais. Lors de l'administration du choc la porte séparant les deux compartiments s'est ouverte, permettant aux animaux de fuir. Lorsque l'animal change de compartiment l'essai est terminé et le choc s'arrête. Si la souris ne change pas de compartiment le choc s'arrête au bout de 24 secondes et l'essai se termine. Ce test a permis d'enregistrer les latences d'échappement des animaux. Les trente essais ont ensuite été groupés par 5 et la moyenne du temps d'échappement a été calculée.

Les molécules, G/A-PE(22-28) et G/A-PE (22-28)-biotinylé ou la solution saline sont injectées 30 minutes avant le conditionnement sous la forme d'un bolus de 100µL d'une solution de 10⁻⁶M préparées dans la solution saline.

Les résultats présentés dans la figure 4 montrent que les peptides G/A-PE(22-28) et G/A-PE(22-28) biotinylé administrés aux souris dans les mêmes conditions que précédemment décrites, sont capables de diminuer les latences d'échappement dans le test de résignation apprise de manière équivalente au peptide PE(22-28).

### EXEMPLE 3 : ACTION DES ANALOGUES DE LA SPADINE SUR LA NEUROGENÈSE ET LA SYNAPTOGENÈSE

### Étude de la neurogenèse par immunohistochimie

Le BrdU (5-bromo-2'-deoxyuridine) est un nucléoside synthétique analogue de la thymidine. Il s'incorpore lors de la réplication de l'ADN, pendant la phase S (duplication des chromosomes lors de l'interphase) des cellules en division. L'injection de BrdU permet de visualiser les cellules en division du tissu à analyser grâce à un marquage immunologique. Dans la présente étude, la neurogenèse hippocampique des souris a été évaluée après traitement par la spadine ou les différents analogues.

Après les tests comportementaux les souris ont été injectées avec du BrdU à une concentration finale de 120 mg/kg en intra-péritonéal, d'après le protocole modifié de Beauquis, J. et al. (Eur J Neurosci 23, 1539-1546, 2006) [25]. Quatre injections de 300 µl à 10 mg/ml, espacées de deux heures, ont été réalisées. 24h après les injections, les souris ont été anesthésiées avec du pentobarbital (100 mg/kg) puis ont été perfusées par voie intracardiaque. Lors de la perfusion intracardiaque, du sérum physiologique a été administré pour éliminer tout le sang de l'organisme des souris, puis du paraformaldéhyde 4% (PFA) a été perfusé pour fixer les organes. Une post-fixation, dans du PFA 4%, a également été réalisée sur la nuit. Les cerveaux des souris ont été coupés à l'aide d'un vibratome (Leica), qui a permis d'obtenir des coupes frontales flottantes de 40 µm. Les coupes sélectionnées pour la réaction immunologique ont été celles allant de bregma 3.3 à 5.3. Huit coupes ont été sélectionnées avec un espacement régulier, ceci permettant de couvrir la totalité de l'hippocampe tout en restant homogène d'un cerveau à l'autre.

La réaction immunologique a été réalisée en incubant un anticorps primaire anti BrdU (BD Pharmingen, 1/8000), avec les coupes de cerveaux, toute une nuit à 4°C. Les coupes de cerveaux ont ensuite été lavées dans du PBS (*Phosphate Buffered Saline*) puis incubées deux heures avec l'anticorps secondaire (anticorps de chèvre anti souris biotinylé, Vecto laboratories, 1/400). Une incubation d'une heure, dans un complexe Avidine/biotine/peroxidase (Kit ABC Vectastain, Vecto laboratories), a étévréalisée dans le but d'amplifier la réaction immunologique. Enfin, la révélation s'est faite à l'aide d'un kit substrat peroxidase (Vector Laboratories) contenant une solution de nickel, une solution 3-3'diaminobenzidine et de l'H₂O₂.

Les coupes ont ensuite été montées sur des lames et fixées avec de l'Entellan. Les cellules positives au BrdU du gyrus denté de l'hippocampe ont été comptées dans les deux hémisphères de chaque cerveau. Le nombre de cellules obtenu sur les 8 coupes a ensuite été multiplié par 40, afin de rendre compte du nombre total de cellules en prolifération dans tout l'hippocampe.

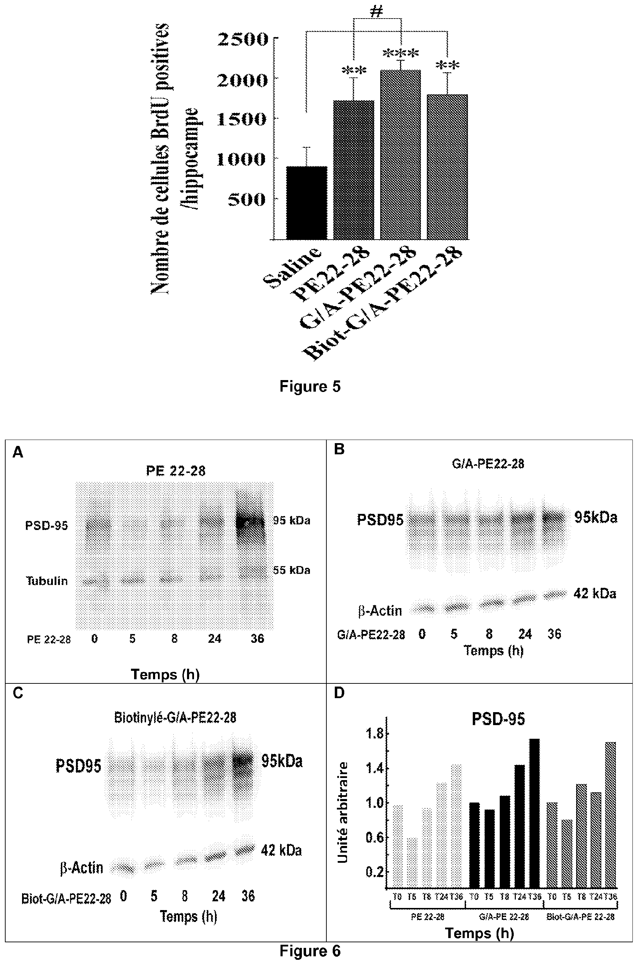

Les résultats présentés dans la figure 5 montrent que les peptides G/A-PE(22-28) et G/A-PE(22-28) biotinylé sont capables d'induire une augmentation de la neurogenèse après seulement quatre jours de traitement. Ces augmentations sont supérieures à l'augmentation obtenue dans les mêmes conditions pour le peptide PE (22-28) non modifié.

### Synaptogenèse

Des souris C57B1/6J femelles gestantes (20-25g, Janvier Labs, St Berthevin, France) ont été anesthésiées par inhalation de 2% isoflurane mélangés avec 30% d'oxygène et 70% d'oxyde nitreux, puis sacrifiés. Les embryons (E14) ont été prélevés et les cortex cérébraux ont été disséqués dans du PBS contenant 1% de glucose. Les neurones ont été préparés à partir des cortex cérébraux par dissociation mécanique, comme décrit dans Brewer et Torricelli, 2007 [26]. Les neurones dissociés ont été répartis dans des plaques traitées à la poly-L-lysine et cultivés jusqu'à 10 jours dans du milieu neurobasal contenant 2% de B27 (Invitrogen, Fisher Scientific, Illkirch, France) et 50 µg/ml de gentamycine (Sigma France), à 37°C sous 5% de CO₂.

Les analogues de la spadine G/A-PE(22-28) et G/A-PE(22-28) biotinylé ont été synthétisés par Covalab,, Villeurbanne, France.

Les cultures de neurones corticaux, isolés à partir de neocortex d'embryons de souris C57BI6/J (Janvier) au stade 14 jours (E14), ont été incubées avec 1 µM de chaque analogue de la spadine. Les incubations ont été réalisées dans le tampon Earle's-Tris-HEPES, à pH 7,4, contenant 140 mM de NaCl, 5 mM de KCI, 1,8 mM de CaCl2, 3,6 mM de MgCl2 et 0,1% de BSA en présence.

Les neurones corticaux traités avec les peptides G/A-PE(22-28) et G/A-PE(22-28) biotinylé pendant différentes durées (0, 5 ,8, 24 et 36 heures) ont été homogénéisés dans du tampon de Laemmli et analysés en utilisant des gels SDS Page 10%. Les protéines séparées ont alors été transférées des gels sur des membranes de nitrocellulose (VWR, Fontenay-sous-bois, France) et bloquées soit avec 5% de lait écrémé soit avec 5% de BSA dans du PBS, pendant 30 minutes, à température ambiante. Les membranes ont été incubées avec des anticorps dirigées contre la protéine PSD-95 (Cell Signaling, Ozyme, Montigny-le-Bretonneux, France), toute la nuit, à 4°C. Les contenus en β-actine ont été déterminés après extraction en utilisant une dilution au 1/5000 d'anticorps anti-β-actin (l'anticorps monoclonal de souris Santa Cruz, dilution 1/5000). Après 4 lavages dans du PBS-Tween 0,1%, des anticorps secondaires anti-souris et anti-lapin conjugués au HRP (Amersham Biosciences, Orsay, France ; 1/10000) sont incubés pendant 1 heure, à température ambiante. Les protéines ont été détectées avec les réactifs de détection ECL Plus (Amersham Biosciences) en utilisant le système d'imagerie LAS-3000 (Fujifilm, Düsseldorf, Allemagne). Les intensités relatives de chaque bande marquée ont été analysées par balayage densitométrique en utilisant le logiciel ImageJ (Wayne Rasband, National Institute of Health, Bethesda, MD, USA). L'activation des protéines a été normalisée en utilisant la β-actine totale comme indiqué.

Les résultats présentés dans la figure 6 montrent que les analogues de la spadine ont rapidement augmenté la synaptogenèse. Les neurones corticaux traités avec les analogues de la spadine G/A-PE(22-28) ou G/A-PE(22-28) biotinylé (1 µM) affichent une augmentation de l'expression de la protéine synaptique PSD-95 observable après 24 heures et de manière plus importante après 36 heures. Il est à noter qu'à l'exception d'une légère baisse durant les 5 premières heures, les traitements avec les analogues de la spadine augmentent continuellement l'expression de PSD-95 jusqu'à 36h (figure 6D). Ces augmentations sont supérieures à l'augmentation obtenue dans les mêmes conditions pour le peptide PE (22-28) non modifié. Ces résultats montrent que les analogues de la spadine G/A-PE(22-28) ou G/A-PE(22-28) biotinylé augmentent plus fortement la neurogenèse que le peptide PE(22-28) mais également plus fortement la synaptogenèse, indiquant que le sort d'une majorité des cellules néoformées est de générer des neurones matures.

### Liste des références

[1] Wong, M. & Licinio, J. Research and treatment approaches to depression. Nat Rev Neurosci. 2, 343-351 (2001).
[2] Moller HJ. Suicide, suicidality and suicide prévention in affective disorders. Acta Psychiatr Scand; 418 (suppl) : 73-80 (2003).
[3] Nestler, E. et al. Neurobiology of depression. Neuron 34, 13-25 (2002).
[4] Baghai TC, Volz HP, Moller HJ. Drug treatment of dépression in the 2000s: An overview of achievements in the last 10 years and future possibilities. World J Biol Psychiatry; 7(4):198-222 (2006).
[5] Weilburg JB. An overview of SSRI and SNRI thérapies for depression. Manag Care. Jun; 13 (6 Suppl Depression): 25-337 (2004).
[6] Manji HK, Gottesman II, Gould TD. Signal transduction and genes-to-behaviors pathways in psychiatric diseases. Sci STKE; 207 : pe49 (2003).
[7] Reus VI, Wolkowitz OM. Antiglucocorticoid drugs in the treatment of depression. Expert Opin Investig Drugs ; 10 : 1789-96 (2001).
[8] Griebel G, Simiand J, Steinberg R, et al. 4-(2-Chloro-4-methoxy-5-methylphenyl)-N-[(1S)-2-cyclopropyl-1-(3- fluoro-4- methylphenyl)ethyl]5-methyl-N-(2-propynyl)-1, 3- thiazol-2-amine hydrochloride (SSR125543A), a potent and sélective corticotrophin-releasing factor(1) receptor antagonist. II. Characterization in rodent models of stress-related disorders. J Pharmacol Exp Ther ; 301 : 333-45 (2002).
[9] Kramer MS, Cutler N, Feighner J, et al. Distinct mechanism for antidepressant activity by blockade of central substance P receptors. Science ; 281 : 1640-1645 (1998).
[10] Skolnick P. Antidepressants for the new millennium. Eur J Pharmacol ; 375 : 31-40 (1999).
[11] Kempermann G, Kronenberg G. Depressed new neurons. Adult hippocampal neurogenesis and a cellular plasticity hypothesis of major depression. Biol Psychiatry ; 54 : 499-503 (2003).
[12] Malberg JE, Schecter LE. Increasing hippocampal neurogenesis: a novel mechanism for antidepressant drugs. Curr Pharm Des; 11 : 145-155 (2005)
[13] Duman, R. & Monteggia, L. A neurotrophic model for stress-related mood disorders. Biol Psychiatry. (9, 1116-1127 (2006).
[14] Henn FA, Vollmayr B. Neurogenesis and depression: etiology or epiphenomenon? Biol Psychiatry; 56 : 146-50.(2004).
[15] Perlis, R. H., Moorjani, P., Fagerness, J., Purcell, S., Trivedi, M. H., Fava, M., Rush, A. J., Smoller, J. W.,. Pharmacogenetic Analysis of Genes Implicated in Rodent Models of Antidepressant Response: Association of TREK1 and Treatment Resistance in the STAR(*)D Study. Neuropsychopharmacology. Nov;33(12):2810-9 (2008)
[16] Jean Mazella, Olivier Petrault, Marc Borsotto, Catherine Heurteaux, Catherine Widmann Peptide dérivé du récepteur 3 de la neurotensine et utilisation dans le traitement de maladies psychiatriques. WO2009103898 A1 ; PCT/FR2008/001784, date de publication 27 août 2009
[17] H. Moha ou Maati, J. Veyssiere, F. Labbal, T. Coppola, C. Gandin, C. Widmann, J. Mazella, C. Heurteaux, M. Borsotto. Spadin as a new antidepressant: absence of side effects on TREK-1 controlled functions. Neuropharmacology. 2012 Jan;62(1):278-88. doi: 10.1016/j.neuropharm.2011.07.019. Epub 2011 Jul 22.
[18] C. Devader, A. Khayachi, J. Veyssière, H. Moha ou Maati, M. Roulot, S. Moreno, M. Borsotto, S Martin, C. Heurteaux and J. Mazella. In vitro and in vivo régulation of synaptogenesis by novel antidepressant spadin. British Journal of Pharmacology. 2015 May;172(10):2604-17. doi: 10.1111/bph.13083. Epub 2015 Mar 24.
[19] Alloui A, Zimmermann K, Mamet J, Duprat F, Noël J, Chemin J, Guy N, Blondeau N, Voilley N, Rubat-Coudert C, Borsotto M, Romey G, Heurteaux C, Reeh P, Eschalier A, Lazdunski M. TREK-1, a K+ channel involved in polymodal pain perception. EMBO J. 2006 Jun 7; 25(11):2368-76.
[20] Krieger DE, Erickson BW, Merrifield RB. Affinity purification of synthetic peptides. Proc Natl Acad Sci USA. 1976 Sep; 73(9):3160-4.
[21] Moha ou Maati, H., Peyronnet, R., Devader, C., Veyssiere, J., Labbal, F., Gandin, C., Mazella, J., Heurteaux, C., Borsotto, M.A. human TREK-1/HEK cell line: a highly efficient screening tool for drug development in neurological diseases. PLoS One. 2011;6(10):e25602. doi: 10.1371/journal.pone.0025602. Epub 2011 Oct 14. (2011).
[22] Nestler, E.J. et al. Preclinical models: status of basic research in depression. Biol Psychiatry. 15, 503-528 (2002).
[23] Cryan, J. & Holmes, A. The ascent of mouse: advances in modelling human dépression and anxiety. Nat Rev Drug Discov. 4, 775-790 (2005).
[24] Seligman and Beagley, Learned helplessness in the rat. J Comp Physiol Psychol 88, 534-541. (1975).
[25] Beauquis, J. et al. Reduced hippocampal neurogenesis and number of hilar neurones in streptozotocin-induced diabetic mice: reversion by antidepressant treatment. Eur J Neurosci 23, 1539-1546. (2006)
[26] Brewer et Torricelli JR. Isolation and culture of adult neurons and neurospheres. Nat Protoc.;2(6):1490-8. (2007).
[27] Hivelin C, Béraud-Dufour S, Devader C, Abderrahmani A, Moreno S, Moha Ou Maati H, Djillani A, Heurteaux C, Borsotto M, Mazella J, Coppola T.(2016) Potentiation of Calcium Influx and Insulin Sécrétion in Pancreatic Beta Cell by the Spécifie TREK-1 Blocker Spadin. J Diabètes Res. 2016;2016:3142175. doi: 10.1155/2016/3142175).

## Revendications

1. Peptide dérivé du propeptide (PE) du récepteur 3 de la neurotensine (NTSR3) choisi parmi le peptide PE(22-28) biotinylé correspondant à la séquence SEQ ID NO : 3 biotinylée, le peptide PI-PE(22-28) de séquence SEQ ID NO : 6 (PIGVSWGLR), le peptide PI-PE(22-28) biotinylé correspondant à la séquence SEQ ID NO : 6 biotinylée, le peptide G/A-PE(22-28) de séquence SEQ ID NO : 7 (AVSWGLR) correspondant à la séquence SEQ ID NO : 3 dans laquelle la glycine en position 22 a été remplacée par une alanine, le peptide G/A-PE(22-28) biotinylé correspondant à la séquence SEQ ID NO : 7 biotinylée, le peptide dansyle-PE(22-28) correspondant à la séquence SEQ ID NO : 3 dans laquelle un groupe chimique dansylique a été ajouté en N-terminal, et les peptides O-méthyl-PE(22-28) et O-éthyl-PE(22-28) correspondant à la séquence SEQ ID NO : 3 dans laquelle un groupe chimique O-méthyl et O-éthyl a été ajouté en C-terminal, respectivement.

2. Séquence d'acide nucléique codant pour un peptide consistant en : le peptide PI-PE(22-28) de séquence SEQ ID NO : 6 (PIGVSWGLR), le peptide PI-PE(22-28) biotinylé correspondant à la séquence SEQ ID NO : 6 biotinylée, le peptide G/A-PE(22-28) de séquence SEQ ID NO : 7 (AVSWGLR) correspondant à la séquence SEQ ID NO : 3 dans laquelle la glycine en position 22 a été remplacée par une alanine, le peptide G/A-PE(22-28) biotinylé correspondant à la séquence SEQ ID NO : 7 biotinylée.

3. Vecteur comprenant une séquence d'acide nucléique selon la revendication 2.

4. Cellule hôte comprenant un peptide selon la revendication 1 et/ou une séquence d'acide nucléique selon la revendication 2 et/ou un vecteur selon la revendication 3.

5. Procédé de production d'un peptide tel que défini dans la revendication 2 comprenant les étapes suivantes :
- transfecter une cellule hôte avec un acide nucléique selon la revendication 2 ou transformer une cellule hôte avec un vecteur selon la revendication 3 ;
- cultiver ladite cellule hôte dans des conditions permettant l'expression du dudit peptide ; et
- récupérer ledit peptide.

6. Peptide selon la revendication 1 pour utilisation comme médicament.

7. Peptide pour utilisation selon la revendication 6, où ledit médicament est un antidépresseur.

8. Peptide selon la revendication 1, pour une utilisation dans le traitement de la dépression.

9. Peptide selon la revendication 1, pour une utilisation dans le traitement du diabète, ou du cancer.

10. Peptide selon la revendication 1, pour une utilisation dans la neuroprotection face à l'épilepsie, ou à l'AVC.

## Patentansprüche

1. Peptid, das von dem Propeptid (PE) des Rezeptors 3 des Neurotensins (NTSR3) abgeleitet ist, ausgewählt aus dem biotinylierten Peptid PE(22-28), das der Sequenz SEQ ID NO: 3 entspricht, dem Peptid PI-PE(22-28) der Sequenz SEQ ID NO: 6 (PIGVSWGLR), dem biotinylierten Peptid PI-PE(22-28), das der Sequenz SEQ ID NO: 6 entspricht, dem Peptid G/A-PE(22-28) der Sequenz SEQ ID NO: 7 (AVSWGLR), das der Sequenz SEQ ID NO: 3 entspricht, in der das Glycin an Position 22 durch ein Alanin ersetzt worden ist, dem biotinylierten Peptid G/A-PE(22-28), das der biotinylierten Sequenz SEQ ID NO: 7 entspricht, dem Peptid Dansyl-PE(22- 28), das der Sequenz SEQ ID NO: 3 entspricht, wobei eine chemische Dansyl-Gruppe an dem N-Terminus hinzugefügt wurde, und den Peptiden O-Methyl-PE(22-28) und O-Ethyl-PE(22-28), die der Sequenz SEQ ID NO: 3 entsprechen, in der eine chemische Gruppe O-Methyl bzw. O-Ethyl an dem C-Terminus hinzugefügt wurde.

2. Nukleinsäuresequenz, die für ein Peptid kodiert, bestehend aus: dem Peptid PI-PE(22-28) der Sequenz SEQ ID NO: 6 (PIGVSWGLR), dem biotinylierten Peptid PI-PE(22-28), das der Sequenz SEQ ID NO: 6 entspricht, dem Peptid G/A-PE(22-28) der Sequenz SEQ ID NO: 7 (AVSWGLR), das der Sequenz SEQ ID NO: 3 entspricht, in der das Glycin an Position 22 durch ein Alanin ersetzt worden ist, dem biotinylierten Peptid G/A-PE(22-28), das der biotinylierten Sequenz SEQ ID NO: 7 entspricht.

3. Vektor, umfassend eine Nukleinsäuresequenz nach Anspruch 2.

4. Wirtszelle, umfassend ein Peptid nach Anspruch 1 und/oder eine Nukleinsäuresequenz nach Anspruch 2 und/oder einen Vektor nach Anspruch 3.

5. Verfahren zur Herstellung eines Peptids wie in Anspruch 2 definiert, umfassend die folgenden Schritte:
- transfizieren einer Wirtszelle mit einer Nukleinsäure nach Anspruch 2 oder Transformieren einer Wirtszelle mit einem Vektor nach Anspruch 3;
- Kultivieren der Wirtszelle unter Bedingungen, die die Expression des Peptids ermöglichen; und
- Gewinnen des Peptids.

6. Peptid nach Anspruch 1 zur Verwendung als Medikament.

7. Peptid zur Verwendung nach Anspruch 6, wobei das Medikament ein Antidepressivum ist.

8. Peptid nach Anspruch 1 zur Verwendung bei der Behandlung von Depressionen.

9. Peptid nach Anspruch 1 zur Verwendung bei der Behandlung von Diabetes, oder Krebs.

10. Peptid nach Anspruch 1 zur Verwendung bei der Neuroprotektion gegen Epilepsie oder Schlaganfall.

## Claims

1. Peptide derived from the propeptide (PE) of the neurotensin receptor 3 (NTSR3) selected from the biotinylated peptide PE(22-28) corresponding to the sequence SEQ ID NO: 3 biotinylated, the peptide PI-PE(22-28) of sequence SEO ID NO: 6 (PIGVSWGLR), the biotinylated peptide PI-PE(22-28) corresponding to the sequence SEQ ID NO: 6 biotinylated, the peptide G/A-PE(22-28) of sequence SEQ ID NO: 7 (AVSWGLR) corresponding to the sequence SEQ ID NO: 3 in which glycine at position 22 was replaced by alanine, the biotinylated peptide G/A-PE(22-28) corresponding to the sequence SEQ ID NO: 7 biotinylated, the peptide dansyl-PE(22-28) corresponding to the sequence SEQ ID NO: 3 in which a dansylic chemical group was added at the N-terminus, and the peptides O-methyl-PE(22-28) and O-ethyl-PE(22-28) corresponding to the sequence SEQ ID NO: 3 in which an O-methyl and O-ethyl chemical group was added at the C-terminus, respectively.

2. Nucleic acid sequence encoding a peptide consisting of: the peptide PI-PE(22-28) of sequence SEQ ID NO: 6 (PIGVSWGLR), the biotinylated peptide PI-PE(22-28) corresponding to the sequence SEQ ID NO: 6 biotinylated, the peptide G/A-PE(22-28) of sequence SEQ ID NO: 7 (AVSWGLR) corresponding to sequence SEQ ID NO: 3 in which the glycine at position 22 was replaced by an alanine, the biotinylated peptide G/A-PE(22-28) corresponding to the sequence SEQ ID NO: 7 biotinylated.

3. Vector comprising a nucleic acid sequence according to claim 2.

4. Host cell comprising a peptide according to claim 1 and/or a nucleic acid sequence according to claim 2 and/or a vector according to claim 3.

5. Method of producing a peptide as defined in claim 2 comprising the following steps:
- transfecting a host cell with a nucleic acid according to claim 2 or transforming a host cell with a vector according to claim 3;
- culturing said host cell under conditions enabling the expression of said peptide; and
- recovering said peptide.

6. Peptide according to claim 1 for use as a medicament.

7. Peptide for use according to claim 6, wherein the medicament is an antidepressant.

8. Peptide according to claim 1 for use in the treatment of depression.

9. Peptide according to claim 1 for use in the treatment of diabetes, or cancer.

10. Peptide according to claim 1 for use in neuroprotection against epilepsy or stroke.
